# EUROPEAN PATENT APPLICATION

(11) **EP 2 239 571 A1**
(43) Date of publication of application: **13.10.2010**
(21) Application number: 09157822.9
(22) Date of filing: 10.04.2009
(51) Int. Cl.: G01N 33/50

(54) **Method for profiling drug compounds using protein kinase inhibitors**

(71) Applicant: PamGene B.V., 5211 's Hertogenbosch (NL)
(72) Inventor: The designation of the inventor has not yet been filed
(74) Representative: De Clercq, Ann G. Y.

(57) **Abstract**

The present invention relates to a method for determining the effect of a drug on the kinase activity in a sample or predicting the response of a patient to a drug, wherein the kinase activity of a sample in the presence of a protein kinase inhibitor is measured, said protein kinase inhibitor mimicking the effect of said drug compound on the kinase activity in said sample.

## Description

### FIELD OF THE INVENTION

The present invention relates to methods for determining the effect of a drug on the kinase activity in a sample or predicting the response of a patient to a drug.

### BACKGROUND OF THE INVENTION

Biological cells contain receptor molecules located on their external membrane. The function of these receptors is to "sense" the cell environment and supply the cell with an input signal about any changes in the environment. In eukaryotic organisms such cell environment is comprised of the neighbouring cells and the function of the receptor is to allow cells to communicate with each other directly or indirectly thus achieving harmonized response of a tissue, organ or a whole organism. In prokaryotic cells, the surface localized receptors provide a means for detecting extracellular environment.

Having received such a signal from for instance neurotransmitters, growth factors, hormones or chemoattractant or chemorepellant substances, the surface localized receptors transmit this information about extracellular environment into the cell through specific intracellular pathways in such a way that the cell responds in the specific fashion to accommodate these changes. These signal transduction pathways are one of the most important areas of investigation in biological research, and involves many types of interactions. One of the major mechanisms frequently employed by cells to regulate their activity, and in particular to regulate signal transduction processes, involves changes in protein phosphorylation. Since protein phosphorylation is involved in numerous regulatory events in cells. For example, specific phosphorylation of various proteins, mediated through phosphorylation by kinases or dephosphorylation by phosphatases, often provides a mechanism through which cell surface signalling pathways transmit and integrate information into the nucleus. Protein phosphorylation commonly occurs on the hydroxy group of an amino acid such as tyrosine, serine, or threonine within a polypeptide, and changes in the phosphorylation state of these polypeptides regulate many aspects of cellular metabolism, growth, and/or differentiation.

Due to the association of the receptor molecules and their signal transduction pathways with various diseases, the receptor molecules are interesting targets for therapeutic intervention, and many clinically useful drugs have been designed or found to act on these receptors, thereby inhibiting or activating the associated signal transduction pathways.

Numerous methods are known for detecting the interaction of the drugs with the receptor. While being very precise and convenient, these methods do not allow to provide information regarding the effect of the drugs on the signal transduction pathways in the cells. For determining the biological activity of the drugs, cell based assays are required. These methods are however very inconvenient because they require very laborious manipulations. Additionally as most of the methods monitor metabolic changes in the cells, they provide a low throughput speed and require the use of live cells that sometimes need immobilization. The methods according to the art therefore show a high variability of the experimental results, and cannot be found to be very trustworthy.

The present invention therefore aims at providing high-throughput and highly sensitive methods and devices for monitoring the cellular response to a specific drug compound on a cell line, laboratory animal or patient thereby assessing the effect of said drug on a cell line, laboratory animal or patient. The present invention also aims to provide methods and devices for predicting the response of a patient to said drug compound.

### SUMMARY OF THE INVENTION

The present invention provides high-throughput and highly sensitive methods and devices that enable the monitoring of the cellular responses of a sample of a patient to a specific drug compound. The measurement methods according to the present invention are based on the measurement of the kinase activity of a sample in the presence of a protein kinase inhibitor said protein kinase inhibitor mimicking the effect of said drug compound on the kinase activity in said cells.

Said drug compounds are biopharmaceutical drug compounds such as antibody drugs, which do not inhibit kinase activity but act on cell surface receptors and/or act on the interaction of said cell surface receptors with signalling molecules such as growth factors, cytokines or hormones.

The present invention therefore provides a method for determining the effect of a drug on the kinase activity in a sample. In a first embodiment of the present invention, the method comprises the steps of:
(a) measuring the kinase activity of said sample, in the presence and in the absence of a protein kinase inhibitor, thereby providing a phosphorylation profile of said sample in the presence of a protein kinase inhibitor and a phosphorylation profile of said sample in the absence of a protein kinase inhibitor; and,
(b) determining from said phosphorylation profiles in the presence and in the absence of a protein kinase inhibitor the differential phosphorylation profile, said differential phosphorylation profile indicating the effect of said drug on the kinase activity in said sample,
wherein said protein kinase inhibitor mimics the effect of said drug on the kinase activity in said sample.

In yet another embodiment the present invention relates to a method for predicting the resistance of a subject to a drug. The method comprises the steps of:
(a) measuring the kinase activity of a sample, obtained from said subject, in the presence and in the absence of a protein kinase inhibitor, thereby providing a phosphorylation profile of said sample in the presence of a protein kinase inhibitor and a phosphorylation profile of said sample in the absence of a protein kinase inhibitor; and,
(b) determining from said phosphorylation profiles in the presence and in the absence of a protein kinase inhibitor the differential phosphorylation profile, said differential phosphorylation profile indicating the effect of said drug on the kinase activity in said sample,
wherein said protein kinase inhibitor reports or indicates the activity of alternative signal transduction pathways causing tumor growth, which are not affected by the drug, thereby causing resistance.

In yet another embodiment, the present invention relates to a method according to the present invention wherein said drug acts on cell surface receptors and/or acts on the interaction of said cell surface receptors with signalling molecules, preferably polypeptides such as for instance growth factors, cytokines and/or hormones.

More preferably said cell surface receptor is a receptor tyrosine kinase, a G-protein coupled receptor or an integrin receptor.

Another embodiment of the present invention relates to a method according to the present invention wherein said kinase substrates carrying phosphorylation sites are located or immobilized on a solid support, and preferably a porous solid support. Preferably said immobilized kinase substrates carrying phosphorylation sites will be immobilized proteins, peptides or peptide mimetics. In a preferred embodiment of the present invention peptides are immobilized on a solid support or a microarray.

Another embodiment of the present invention regards a according to the methods of the present invention wherein said differential phosphorylation profile predicts the clinical outcome of a drug therapy.

These and further aspects and embodiments are described in the following sections and in the claims.

### BRIEF DESCRIPTION OF FIGURES

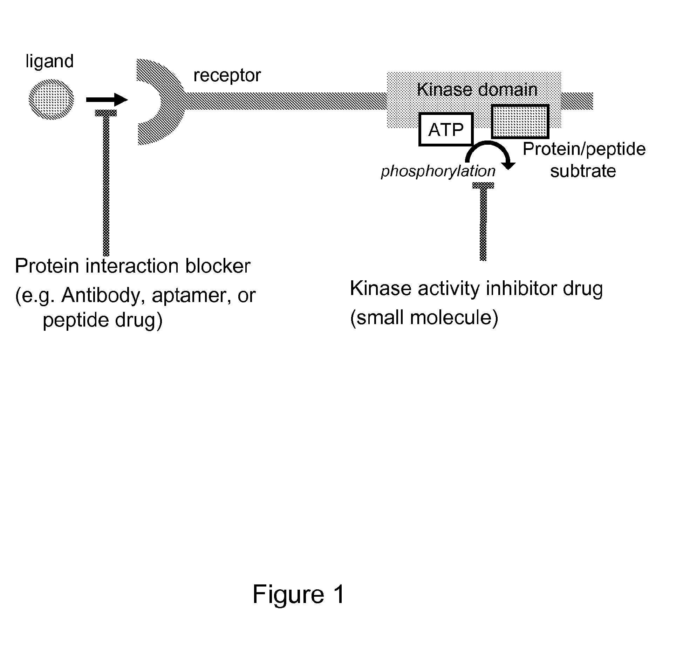

**Figure 1** provides a representation of a receptor molecule and the transmission of information towards the intracellular signal transduction pathways.

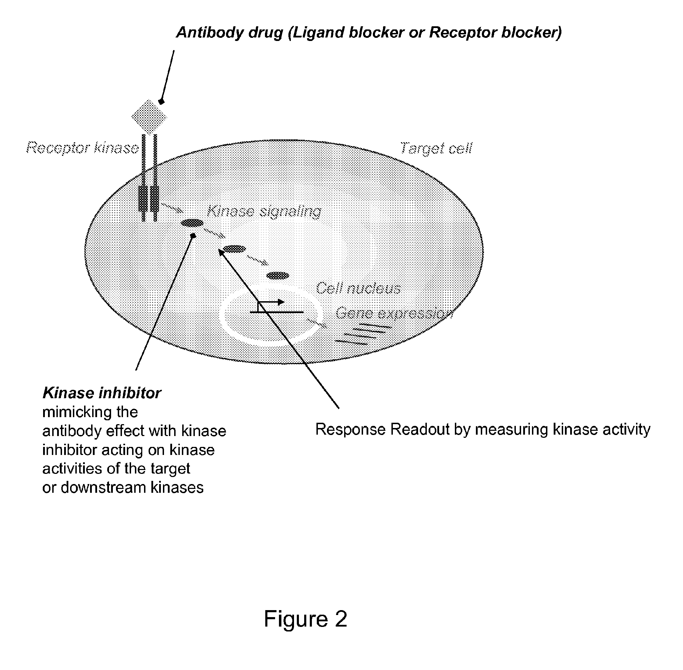

**Figure 2** provides a representation showing how the effect of biopharmaceutical drug compounds, acting on cell surface receptors and/or acting on the interaction of said cell surface receptors with signalling molecules, can be mimicked in the methods of the present invention by using protein kinase inhibitors acting against the protein kinases that provide the first steps in the signal transduction pathway associated with said cell surface receptors.

### DETAILED DESCRIPTION OF THE INVENTION

Before the present method and devices used in the invention are described, it is to be understood that this invention is not limited to particular methods, components, or devices described, as such methods, components, and devices may, of course, vary. It is also to be understood that the terminology used herein is not intended to be limiting, since the scope of the present invention will be limited only by the appended claims.

Unless defined otherwise, all technical and scientific terms used herein have the same meaning as commonly understood by one of ordinary skill in the art to which this invention belongs. Although any methods and materials similar or equivalent to those described herein may be used in the practice or testing of the present invention, the preferred methods and materials are now described.

In this specification and the appended claims, the singular forms "a", "an", and "the" include plural references unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps.

The terms "comprising", "comprises" and "comprised of" also include the term "consisting of".

The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

The recitation of numerical ranges by endpoints includes all numbers and fractions subsumed within the respective ranges, as well as the recited endpoints.

The present invention provides high-throughput and highly sensitive methods and devices that enable the monitoring of the cellular responses of cells to a specific drug compound. The measurement methods according to the present invention are based on the measurement of the kinase activity of a cell sample of a patient in the presence of a protein kinase inhibitor said protein kinase inhibitor mimicking the effect of said drug compound on the kinase activity in said cells.

Said drug compounds are biopharmaceutical drug compounds such as antibody drugs, which do not inhibit kinase activity but act on cell surface receptors (Figure 1) and/or act on the interaction of said cell surface receptors with signalling molecules such as growth factors, cytokines or hormones.

This incompatibility is due to the fact that such protein interaction blockers like antibodies such as Herceptin, do not act by inhibiting kinase activity,

Preferably, in one embodiment of the present invention, methods are provided wherein the kinase activity is protein kinase activity. For purposes of the present invention, and as used herein the term "enzyme activity", "kinase activity" or "protein kinase activity" refer to the amount of reaction product(s) that a certain amount of enzyme, kinase or protein kinase acting on a substrate will produce in a specified period of time.

Protein kinase activity is referred to as the activity of protein kinases. A protein kinase is a generic name for all enzymes that transfer a phosphate to a protein. About three to four percent of the human genome contains transcription information for the formation of protein kinases. Currently, there are about 518 known different protein kinases. However, because three to four percent of the human genome is a code for the formation of protein kinases, there may be many more separate kinases in the human body.

A protein kinase is a kinase enzyme that modifies other proteins by covalently coupling phosphate groups to them. This process or activity is also referred to as phosphorylation. Phosphorylation can therefore be regarded as the process of the addition of a phosphate group to a substrate. Phosphorylation usually results in a functional change of the substrate by changing enzyme activity, cellular location, or association with other proteins. Up to 30% of all proteins may be modified by kinase activity, and kinases are known to regulate the majority of cellular pathways, especially those involved in signal transduction, the transmission of signals within the cell. The chemical activity of a kinase involves removing a phosphate group from ATP or GTP and covalently attaching it to amino acids such as serine, threonine, tyrosine, histidine, aspartic acid and/or glutamic acid that have a free hydroxyl group. Most known kinases act on both serine and threonine, others act on tyrosine, and a number act on all serine, threonine and tyrosine. The protein kinase activity monitored with the method of the present invention is preferably directed to protein kinases acting towards serine, threonine and/or tyrosine, preferably acting on both serine and threonine, on tyrosine or on serine, threonine and tyrosine and more preferably the method of the present invention if preferably directed to protein kinases acting towards tyrosines.

Protein kinases are distinguished by their ability to phosphorylate substrates on discrete sequences. These sequences have been determined by sequencing the amino acids around the phosphorylation sites and are usually distinct for each protein kinase. The recognition sequence on each substrate is for a specific kinase catalyst.

Because protein kinases have profound effects on a cell, their activity is highly regulated. Kinases are turned on or off by for instance phosphorylation, by binding of activator proteins or inhibitor proteins, or small molecules, or by controlling their location in the cell relative to their substrates. Deregulated kinase activity is a frequent cause of disease, particularly cancer, where kinases regulate many aspects that control cell growth, movement and death. Therefore monitoring the protein kinase activity in tissues can be of great importance and a large amount of information can be obtained when comparing the kinase activity of different tissue samples.

As described in the present invention, the inventors have surprisingly found that the effect of biopharmaceutical drug compounds, acting on cell surface receptors and/or acting on the interaction of said cell surface receptors with signalling molecules, can be mimicked in the methods of the present invention by using protein kinase inhibitors acting against the protein kinases that provide the first steps in the signal transduction pathway associated with said cell surface receptors (Figure 2).

When added to samples comprising cells of said patient said biopharmaceutical drug compounds may act on cell surface receptors and inhibit the interaction of signalling molecules with said cell surface receptors. Consequently, said cell surface receptor will not activate the associated signal transduction pathway. The effect of said biopharmaceutical drug compounds can thus be monitored by comparing samples comprising cells treated with said drug with samples comprising non-treated cells. However, as the biopharmaceutical drug compounds act on cell surface receptors, it is required that the cells remain intact and one monitors the metabolic changes in the cells. Additionally it may be required that the cells need immobilization. Measuring the effect of the biopharmaceutical drug compounds is thus a very cumbersome method.

The inventors have found that treating a cell sample of a patient, such as a cell lysate, with a protein kinase inhibitor inhibiting one of the first steps in the signal transduction pathway associated with a cell surface receptor, and measuring the effect of said protein kinase inhibitor on the signal transduction pathway, enables the association of said effect with biopharmaceutical drug compounds acting on said cell surface receptor. Said protein kinase inhibitor actually mimicks the biopharmaceutical drug compounds and provides a much easier method for measuring the effect of the biopharmaceutical drug compounds. The measurement can be performed on cell lysates and does not require the very cumbersome incubation of said biopharmaceutical drug compounds with live cells.

As referred to in the present invention "mimicking" refers to the aspect of the invention where the effects caused by the protein kinase inhibitors used in the methods of the present invention can be associated to the effects caused by the biopharmaceutical drug compounds. Accordingly, as the effect of a protein kinase inhibitor on a sample can be associated to the effect of a biopharmaceutical drug compound, said protein kinase inhibitor is used in the methods of the present invention to provide information regarding the effect of a biopharmaceutical drug compound, without having to use said biopharmaceutical drug compound. The protein kinase inhibitor may therefore act towards a cell surface receptor which is targeted by the biopharmaceutical drug compound. Said protein kinase inhibitor may also act towards cell surface receptor associated proteins, and preferably proteins associated with the signal transduction pathway of said cell surface receptor. Said cell surface receptor associated proteins may be kinase and/or receptor proteins preferably signal transduction proteins downstream from said cell surface receptor.

In another embodiment said protein kinase inhibitors may act towards a cell surface receptor or cell surface receptor associated proteins of an alternative signal transduction pathway. As tumor resistance to treatments with specific drug compounds is often caused by alternative signal transduction pathways which are not affected by the drug compounds and cause tumor growth. Therefore the use of protein kinase inhibitors acting towards alternative signal transduction pathways can predict whether a patient would be resistant to said drug.

The measurement of the kinase activity is performed by contacting a sample with one or more substrates, preferably protein kinase substrates, thereby generating a phosphorylation profile.

Said protein kinase substrates as used herein, are preferably peptides, proteins or peptide mimetics. The protein kinase substrates each comprise one or more phosphorylation sites that can be phosphorylated by the protein kinases present in the sample. Therefore, during the measurement method the kinase enzymes actively present in the sample will phosphorylate one or more of the phosphorylation sites on one or more protein kinase substrates. The inventors have observed that differences in the kinase activity cell lysates in the presence and in the absence of a kinase inhibitor is indicative for the effect of a biopharmaceutical drug compound acting on a cell surface receptor, wherein said kinase inhibitor is associated with said cell surface receptor.

The present invention therefore provides a method for determining the effect of a drug on the kinase activity in a sample. In a first embodiment of the present invention, the method comprises the steps of:
(a) measuring the kinase activity of said sample, in the presence and in the absence of a protein kinase inhibitor, thereby providing a phosphorylation profile of said sample in the presence of a protein kinase inhibitor and a phosphorylation profile of said sample in the absence of a protein kinase inhibitor; and,
(b) determining from said phosphorylation profiles in the presence and in the absence of a protein kinase inhibitor the differential phosphorylation profile, said differential phosphorylation profile indicating the effect of said drug on the kinase activity in said sample,
wherein said protein kinase inhibitor mimics the effect of said drug on the kinase activity in said sample.

More preferably the method according to the present invention determines the effect of a drug on the kinase activity in cells or tissues, comprising the steps of:
(a) measuring the kinase activity of a cell lysate, in the presence and in the absence of a protein kinase inhibitor, thereby providing a phosphorylation profile of said sample in the presence of a protein kinase inhibitor and a phosphorylation profile of said sample in the absence of a protein kinase inhibitor; and,
(b) determining from said phosphorylation profiles in the presence and in the absence of a protein kinase inhibitor the differential phosphorylation profile, said differential phosphorylation profile indicating the effect of said drug on the kinase activity in said cell lysate,
wherein said protein kinase inhibitor mimics the effect of said drug on the kinase activity in said cells or tissues.

It should be noted that the methods of the present invention enable to determine the effect of a drug on the kinase activity in cells or tissues by measuring kinase activity of a cell lysate, in the presence and in the absence of a protein kinase inhibitor. The effect of said drug on cells or tissues can be determined by assessing the kinase activity in a sample derived from said cells or tissues, and preferably a lysed sample thereof.

As used in the present invention, the term "sample" refers to a sample obtained from an organism (patient) such as a human or from components (e.g. tissue or cells) of such an organism. Said sample is preferably obtained from a patient and needs to be derived from the tumor tissue of said patient. More preferably said sample is a tumor tissue biopsy, vacuum assisted biopsy, fine needle biopsy or material from a resected tumor. Said sample is thereby referred to as a 'clinical sample' which is a sample derived from a patient.

Said tumor tissue sample is preferably a fresh or a fresh frozen sample.

More preferably, said sample refers to a lysate of a tumor tissue obtained through tumor tissue biopsy, fine needle biopsy or material from a resected tumor. Alternatively said sample may be obtained from specific tumor cell lines and in particular cell lysates thereof.

Alternatively said sample may be derived from a tumor sample that has been cultured in vitro for a limited period of time.

The tissue samples may also refer to surrogate tissues. The ideal tissue to perform pharmacodynamic studies is the own tumor. However, taking in consideration the difficulties to perform sequential tumor biopsies, surrogate tissues can be used instead. Therefore, a distant tissue, such as skin tissue, can be used as a surrogate tissue for a cancerous tissue. The surrogate tissue can be used to monitor, or predict the effects of a drug. For example skin and hair tissue are known for their use as a prediction for the response of tumors to treatment with signalling inhibitors.

In a preferred embodiment of the present invention said sample is a sample that has undergone a preparation step prior to the steps according to the method of the present invention. Preferably said preparation step is a step where the protein kinases present in said sample are released from the tissue by lysis. Additionally the kinases in the sample may be stabilized, maintained, enriched or isolated, and the measurement of the kinase activity as performed in step (a) occurs on the enriched or isolated protein kinase sample. By first enriching protein kinases in the sample or isolating protein kinases from the sample the subsequent measurement of the kinase activity will occur in a more efficient and reliable manner. Also the clarity and intensity of the obtained phosphorylation signal will be increased as certain contaminants are being removed during the enriching or isolating step.

As used in the present invention, the term "phosphorylation profile" refers to a data set representative for the phosphorylation levels of one or more phosphorylation sites present on each protein kinase substrate. When measuring the kinase activity of a sample by contacting said sample with protein kinase substrates a specific phosphorylation profile is obtained. The phosphorylation profile is generated by the phosphorylation of the protein kinase substrates with the protein kinases present in the sample and it comprises the level of phosphorylation of the phosphorylation sites present on the protein kinase substrates used. A phosphorylation profile can thus be generated when using at least one protein kinase substrate in different test conditions such as for example by comparing the phosphorylation level of a sample on one peptide or protein (protein kinase substrate) in the presence and absence of a protein kinase inhibitor. More frequently phosphorylation profiles of a sample will be measured using several protein kinase substrates in the same or sequentially carried out experiments.

It should be noted that a person skilled in the art will appreciate that the methods of the present invention can use phosphorylation profiles as a basis for determining the effect of a drug on the kinase activity or as a basis for predicting the response to a medicament. However, the phosphorylation levels of individual protein kinase substrates can also be used as a basis for determining the effect of a drug on the kinase activity or as a basis for predicting the response to a medicament.

It should be noted that for the measurement of the protein kinase activity, ATP or any other phosphate source needs to be added to the sample when it is contacted with the protein kinase substrates. The presence of ATP will lead to a phosphorylation of the protein kinase substrates. Alternatively, the phosphorylation of the protein kinase substrates can be performed in the absence of exogenous ATP. When no ATP is added during the incubation of the sample with the protein kinase substrates, the endogenous ATP, the ATP naturally present in the sample, will act as the primary source of ATP.

The phosphorylation level of each of the protein kinase substrates can be monitored using any method known in the art. The response of the protein kinase substrates is determined using a detectable signal, said signal resulting from the interaction of the sample with the protein kinase substrates or by for instance measuring mass differences using mass spectrometry. In determining the interaction of the sample with the protein kinase substrates the signal is the result of the interaction of the phosphorylated substrates with a molecule capable of binding to the phosphorylated substrates. This binding can be detected by e.g. surface plasmon resonance or by the molecule being detectably labelled. For the latter, the molecule that specifically binds to the substrates of interest (e.g. antibody or polynucleotide probe) can be detectably labelled by virtue of containing an atom (e.g. radionuclide), molecule (e.g. fluorescein), or enzyme or particle or complex that, due to a physical or chemical property, indicates the presence of the molecule. A molecule may also be detectably labelled when it is covalently bound to or otherwise associated with a "reporter" molecule (e.g. a biomolecule such as an enzyme) that acts on a substrate to produce a detectable atom, molecule or other complex.

Detectable labels suitable for use in the present invention include any composition detectable by spectroscopic, photochemical, biochemical, immunochemical, electrical, optical or chemical means. Labels useful in the present invention include biotin for staining with labelled avidin or streptavidin conjugate, magnetic beads (e.g. Dynabeads'), fluorescent dyes (e.g. fluorescein, fluorescein-isothiocyanate (FITC), Texas red, rhodamine, green fluorescent protein, enhanced green fluorescent protein, lissamine, phycoerythrin, Cy2, Cy3, Cy3.5, Cy5, Cy5.5, Cy7, FluorX [Amersham], SYBR Green I & II [Molecular Probes], and the like), radiolabels (e.g. 3H, 125I, 35S, 14C, or 32P), enzymes (e.g. hydrolases, particularly phosphatases such as alkaline phosphatase, esterases and glycosidases, or oxidoreductases, particularly peroxidases such as horse radish peroxidase, and the like), substrates, cofactors, inhibitors, chemilluminescent groups, chromogenic agents, and colorimetric labels such as colloidal gold or coloured glass or plastic (e. g. polystyrene, polypropylene, latex, etc.), protein particles or beads.

Means of detecting such labels are well known to those of skill in the art. Thus, for example, chemiluminescent and radioactive labels may be detected using photographic film or scintillation counters, and fluorescent markers may be detected using a photodetector to detect emitted light (e.g. as in fluorescence-activated cell sorting). Enzymatic labels are typically detected by providing the enzyme with a substrate and detecting a coloured reaction product produced by the action of the enzyme on the substrate. Colorimetric labels are detected by simply visualizing the coloured label. Thus, for example, where the label is a radioactive label, means for detection include a scintillation counter, photographic film as in autoradiography, or storage phosphor imaging. Where the label is a fluorescent label, it may be detected by exciting the fluorochrome with the appropriate wavelength of light and detecting the resulting fluorescence. The fluorescence may be detected visually, by means of photographic film, by the use of electronic detectors such as charge coupled devices (CCDs) or photomultipliers and the like. Similarly, enzymatic labels may be detected by providing the appropriate substrates for the enzyme and detecting the resulting reaction product. Also, simple colorimetric labels may be detected by observing the colour associated with the label. Fluorescence resonance energy transfer has been adapted to detect binding of unlabeled ligands, which may be useful on arrays.

In a particular embodiment of the present invention the response of the protein kinase substrates to the sample is determined using detectably labelled antibodies; more in particular fluorescently labelled antibodies. In those embodiments of the invention where the substrates consist of protein kinase substrates, the response of the protein kinase substrates is determined using fluorescently labelled anti-phosphotyrosine antibodies, fluorescently labelled anti-phosphoserine or fluorescently labelled anti-phosphothreonine antibodies. The use of fluorescently labelled anti-phosphotyrosine antibodies or fluorescently labelled anti-phosphoserine or fluorescently labelled anti-phosphothreonine antibodies in the method of the present invention, allows real-time or semi real-time determination of the protein kinase activity and accordingly provides the possibility to express the protein kinase activity as the initial velocity of protein kinase derived from the activity over a certain period of incubation of the sample on the protein kinase substrates.

The term "differential phosphorylation level" as used herein therefore refers to a data set comprising comparison data from the phosphorylation profiles in the presence and in the absence of a protein kinase inhibitor. The statistical analysis of the differential phosphorylation level can be done using multivariate and/or univariate statistical methods known in the art and for instance, but not limited to, using a student t-test. The differential phosphorylation levels are obtained by (numerically) comparing the peptide phosphorylation levels or profiles in the presence and in the absence of the protein kinase inhibitor in the same sample, for instance, but not limited to, providing ratios or differences of the profiles obtained in the presence and the absence of the protein kinase inhibitor.

In addition, because the differential phosphorylation level is generated by comparing the phosphorylation levels or profiles of the same sample in the presence and the absence of the protein kinase inhibitor, preferably during a parallel series of measurements run in the same instrument, the differential phosphorylation level is surprisingly found to be less affected by variation, for example biological variation, experimental variation, compared to single phosphorylation levels or profiles. This provides a more robust, more sensitive, more reproducible and more reliable method for determining the effect of a drug on the kinase activity.

Another embodiment according to the present invention comprises the steps of:
(a) measuring the kinase activity of said sample, in the presence and in the absence of a protein kinase inhibitor, thereby providing a phosphorylation profile of said sample in the presence of a protein kinase inhibitor and a phosphorylation profile of said sample in the absence of a protein kinase inhibitor; and,
(b) determining from said phosphorylation profiles in the presence and in the absence of a protein kinase inhibitor the differential phosphorylation profile, said differential phosphorylation profile indicating the effect of said drug on the kinase activity in said sample,
wherein said protein kinase inhibitor mimics the effect of said drug on the drug targeted signal transduction pathway in said sample.

Said protein kinase inhibitor may therefore act towards a cell surface receptor or cell surface receptor associated proteins, and preferably proteins associated with the signal transduction pathway of said cell surface receptor. Said cell surface receptor associated proteins may be kinase and/or receptor proteins preferably signal transduction proteins downstream from said cell surface receptor.

In yet another embodiment the present invention relates to a method for predicting the resistance of a subject to a drug. The method comprises the steps of:
(a) measuring the kinase activity of a sample, obtained from said subject, in the presence and in the absence of a protein kinase inhibitor, thereby providing a phosphorylation profile of said sample in the presence of a protein kinase inhibitor and a phosphorylation profile of said sample in the absence of a protein kinase inhibitor; and,
(b) determining from said phosphorylation profiles in the presence and in the absence of a protein kinase inhibitor the differential phosphorylation profile, said differential phosphorylation profile indicating the effect of said drug on the kinase activity in said sample,
wherein said protein kinase inhibitor reports or indicates the activity of alternative signal transduction pathways causing tumor growth, which are not affected by the drug, thereby causing resistance.

In yet another embodiment the present invention relates to a method for predicting the resistance of a subject to a drug. The method comprises the steps of:
(a) measuring the kinase activity of said sample, obtained from said subject, in the presence and in the absence of a protein kinase inhibitor, thereby providing a phosphorylation profile of said sample in the presence of a protein kinase inhibitor and a phosphorylation profile of said sample in the absence of a protein kinase inhibitor; and,
(b) determining from said phosphorylation profiles in the presence and in the absence of a protein kinase inhibitor the differential phosphorylation profile, said differential phosphorylation profile indicating the effect of said drug on the kinase activity in said sample,
wherein said protein kinase inhibitor reports or indicates the activity of a signal transduction pathway which is not affected by said drug, thereby reporting or indicating resistance.

Another embodiment according to the present invention relates to a method for predicting the response of a subject to a drug, wherein the effect of a drug on the kinase activity in a sample, obtained from said subject, is determined according to the methods of the present invention, and wherein the determination of the effect of said drug on the kinase activity in said sample, indicates the response of said subject to said drug.

In another embodiment according to the present invention, the phosphorylation profile comprises the phosphorylation levels of, preferably one or more, phosphorylation site(s) present in at least one peptide marker.

The term "peptide markers" in the context of the present invention refers to the fact that peptides can be preferably used according to the methods of the present invention as target regions to measure the phosphorylation levels of phosphorylation sites of said markers in the presence of protein kinase present in samples. The phosphorylation levels of the individual phosphorylation sites present in said markers may be measured and compared in different ways.

The protein kinase substrates as used in the methods described herein, are meant to include peptides, proteins or peptide mimetics comprising one or more of the phosphorylation sites. Said one or more phosphorylation sites are specifically phosphorylated by the protein kinases present in the sample thereby providing a phosphorylation profile. More preferably the protein kinase substrates (peptides, proteins or peptide mimetics) as used in the method of the present invention comprise one or more of the phosphorylation sites

In yet another embodiment, the present invention relates to a method according to the present invention wherein said drug acts on cell surface receptors and/or acts on the interaction of said cell surface receptors with signalling molecules, preferably polypeptides such as for instance growth factors, cytokines and/or hormones.

More preferably said cell surface receptor is a receptor tyrosine kinase, a G-protein coupled receptor or an integrin receptor.

As used herein, the term "receptor tyrosine kinase" refers to high affinity cell surface receptors for many polypeptide growth factors, cytokines and hormones. Receptor tyrosine kinases have been shown to be not only key regulators of normal cellular processes but also to have a critical role in the development and progression of many types of cancer. The receptor function is involved in the transduction of the incoming signal (e.g. growth factor presence) over the membrane initiating the triggering of a signal transduction pathway. This pathway involves multiple phosphorylation events leading to increased activities of kinase members of these pathways. In case of cancer the proliferation and cell division controlling pathways are overactive and cause tumor growth and metastasis. Blocking of the receptors can be done in multiple ways, e.g. by blocking the interaction and binding of the growth factor to the receptor using antibodies that bind to either interaction sites on the growth factor or receptor. Alternative approaches block the kinase activity of the receptor tyrosine kinase, e.g. using small molecular compounds. Resistance to such pharmacotherapies may occur. This can be due to the fact that receptor blocking by antibodies is not effectively preventing activation of the kinase domain of the receptor, or because more downstream signalling events are still not prevented because of mutations, e.g. leading to constitutively active signalling downstream of the targeted tyrosine kinase receptor.

In yet another embodiment, the present invention relates to a method according to the present invention wherein said protein kinase inhibitor is chosen from the group comprising ErbB2 kinase inhibitor such as Lapatinib, mimicking the effect of drugs acting on a HER2/Neu receptor such as Herceptin; VEGFR kinase inhibitor such as Sorafinib, mimicking the effect of drugs acting on VEGF or a VEGF receptor such as Avastin; or a EGFR kinase inhibitor such as Erlotinib or Gefitinib, mimicking the effect of drugs acting on a EGF receptor such as Erbitux; or an IGFR kinase inhibitor, mimicking the effect of drugs on an IGF receptor; an PDGFR kinase inhibitor, mimicking the effect of drugs on an PDGF receptor; or a HGFR (cMET) kinase inhibitor, mimicking the effect of drugs on an HGF receptor.

According to another embodiment, the present invention relates to the method of the present invention wherein said phosphorylation profile, differential phosphorylation profile or level or said classifier parameter indicates or is specific for a certain pathology. Potential pathologies include, but are not limited to, oncological diseases, metabolic diseases, immunological and auto-immunological diseases, diseases of the nervous system and/or infectious diseases.

In another embodiment, the present invention regards the method according to the present invention wherein said differential phosphorylation profile is used for diagnostical and/or prognostical purposes, the prediction of the clinical outcome of a therapy, and/or the prediction of side effects and/or toxic effects and/or adverse effects of a therapy. For example the method of the present invention can be used to diagnose a cancer and preferably brain cancer, thereby differentiating between benign and malignant tumors.

More preferably the present invention relates to a method according to the present invention wherein said differential phosphorylation profile predicts the response of cells, tissues, organs and/or warm-blooded animals to said drug.

As used herein, the term "protein kinase inhibitor" refers to a type of enzyme inhibitor which specifically blocks the action of one or more protein kinases, hence they can be subdivided or characterised by the amino acids whose phosphorylation is inhibited. Examples of protein kinase inhibitors for use in the method of the present invention are Bevacizumab, BIBW 2992, Cetuximab, Imatinib, Trastuzumab, Gefitinib, Ranibizumab, Pegaptanib, Sorafenib, Dasatinib, Sunitinib, Erlotinib, Nilotinib, Lapatinib, Panitumumab, Vandetinib, E7080, imatinib, temsirolimus, ABT-869, AEE788, Alvocidib, AP23464, AP23846, AP23848, ARRY-142886, ARRY-334543, AT-7519, Axitinib, AZD0530, AZD1152, BIRB-796, BMI-1026, BMS-599626, Bosutinib, Brivanib, Canertinib, CCT129202, Cediranib, CEP-7055, CP-547632, CP-724714, Dovitinib, Enzastaurin, everolimus, FI-700, Gossypol, HKI-272, HMN-176, HMN-214, INNO-406, JNJ-7706621, KRX-0601, LBW242, Lestaurtinib, Midostaurin, MK-0457, MLN8054, MP-470, Neratinib, ON0123380, ON01910, ON-01910, OSI-930, Pazopanib, PD166326, PD173955, PD180970, Pelitinib, PF-2341066, PHA665752, PHA-739358, PX-866, R-547, Seliciclib, Semapimod, Semaxanib, SNS-032, SU011248, SU014813, SU11248, SU11274, SU14813, Tandutinib, Telatinib, TSU-68, UCN-01, Vandetanib, Vatalanib, VE-465, ZM 447439, Tyrphostin 1, Tyrphostin 23, Tyrphostin 51, Tyrphostin 63, Tyrphostin A9, Tyrphostin AG 1007, Tyrphostin AG 1112, Tyrphostin AG 1433, Tyrphostin AG 370, Tyrphostin AG 494, Tyrphostin AG 658, Tyrphostin AG 808, Tyrphostin AG 825, Tyrphostin AG 835, Tyrphostin AG 879, Tyrphostin AG 957, Tyrphostin AG 974, Tyrphostin AG 1296, Tyrphostin AG 1478, Tyrphostin AG 490, Tyrphostin RG 13022, Tyrphostin RG 14620, Tyrphostin SU 1498, I-OMe-Tyrphostin AG 538, Protein Kinase G inhibitor peptide (Arg-Lys-Arg-Ala-Arg-Lys-Glu), Geldanamycin, Lavendustin A and/or Genistein. More preferably for the purpose of the present invention, said protein kinase inhibitors are protein kinase inhibitors chosen from the group comprising Lapatinib, Erlotinib, Sorafenib, Sunitinib and/or Gefitinib.

Another embodiment of the present invention relates to a method according to the present invention wherein said kinase substrates carrying phosphorylation sites are located or immobilized on a solid support, and preferably a porous solid support. Preferably said immobilized kinase substrates carrying phosphorylation sites will be immobilized proteins, peptides or peptide mimetics. In a preferred embodiment of the present invention peptides are immobilized on a solid support, which may be a microarray.

As used herein "peptide" refers to a compound generally consisting of 2 to 30 naturally occurring or synthetic amino acids which can also be further modified including covalently linking the peptide bonds of the alpha carboxyl group of a first amino acid and the alpha amino group of a second amino acid by eliminating a molecule of water. The amino acids can be either those naturally occurring amino acids or chemically synthesized variants of such amino acids or modified forms of these amino acids which can be altered from their basic chemical structure by addition of other chemical groups which can be found to be covalently attached to them in naturally occurring compounds.

As used herein "protein" refers to an organic compound made of amino acids arranged in a linear chain and joined together by peptide bonds between the carboxyl and amino groups of adjacent amino acid residues.

As used herein "peptide mimetics" refers to organic compounds which are structurally similar to peptides. The peptide mimetics are typically designed from existing peptides to alter the molecules characteristics. Improved characteristics can involve, for example improved stability such as resistance to enzymatic degradation, or enhanced biological activity, improved affinity by restricted preferred conformations and ease of synthesis. Structural modifications in the peptidomimetic in comparison to a peptide, can involve backbone modifications as well as side chain modification.

For measuring the kinase activity of the sample a large variety of methods and formats are known in the art. The kinase activity can for example be measured using ELISA and multiplex ELISA techniques, blotting methods, mass spectrometry, capillary electrophoresis, bead arrays, macroarrays, microarrays or any other method known in the art. Depending on the type of kinase activity measurement method the solid support on which the proteins, peptides or peptide mimetics may vary. Whereas in ELISA the protein kinase substrates are attached to the walls of the microtiterplates, in microarrays the protein kinase substrates are immobilized on the microarray substrate.

In a preferred embodiment of the present invention the protein kinase substrates are immobilized on an array, and preferably a microarray of protein kinase substrates wherein the protein kinase substrates are immobilized onto a solid support or another carrier. The immobilization can be either the attachment or adherence of two or more protein kinase substrate molecules to the surface of the carrier including attachment or adherence to the inner surface of said carrier in the case of e.g. a porous or flow-through solid support.

In a preferred embodiment of the present invention, the array of protein kinase substrates is a flow-through array. The flow-through array as used herein could be made of any carrier material having oriented through-going channels as are generally known in the art, such as for example described in PCT patent publication WO 01/19517. Typically the carrier is made from a metal oxide, glass, silicon oxide or cellulose. In a particular embodiment the carrier material is made of a metal oxide selected from the group consisting of zinc oxide, zirconium oxide, tin oxide, aluminium oxide, titanium oxide and thallium, in a more particular embodiment the metal oxide consists of aluminium oxide.

Accordingly, in a further embodiment of the present invention said array is a Pamchip®.

In a further embodiment, the present invention relates to a method according to the present invention wherein said solid support (microarray) comprises one ore more peptides immobilized thereto.

Another embodiment of the present invention regards a according to the methods of the present invention wherein said differential phosphorylation profile predicts the clinical outcome of a drug therapy.

By measuring the kinase activity of a sample, in the presence and in the absence of a protein kinase inhibitor, the effect of that protein kinase inhibitor and the associated biopharmaceutical drug compound can be assessed. This method was found particularly useful in the prediction of response to said biopharmaceutical drug compound, and to enable the distinction between responders and non-responders in the treatment with said biopharmaceutical drug compound.

The biopharmaceutical drug compound as used in the method of the present invention can be any kind of chemical substance for instance used in the treatment, cure, prevention, or diagnosis of disease or used to otherwise enhance physical or mental well-being. Specifically said biopharmaceutical drug compound are compounds such as antibody drugs, which do not inhibit kinase activity but act on cell surface receptors and/or act on the interaction of said cell surface receptors with signalling molecules such as growth factors, cytokines or hormones.

In another embodiment of the present invention the method for predicting the clinical outcome of a drug therapy, uses phosphorylation profiles which comprise the phosphorylation levels of one or more phosphorylation sites present in one or more peptide markers. Preferably also this method will use two or more of said peptide markers as described above.

Another embodiment of the present invention regards the use of the method according to the present invention for assessing susceptibility to a drug of a biological species having a specific disease state or cellular condition.

Another embodiment of the present invention regards the use of the method according to the present invention to a potential kinase inhibitor of a biological species having a specific disease state or cellular condition.

Another embodiment of the present invention regards the use of the method according to the present invention for assessing the pharmaceutical value of a drug.

Another embodiment of the present invention regards the use of the method according to the present invention for assessing the clinical value of a drug.

As used herein when assessing susceptibility to a drug, the pharmaceutical value of a drug or the clinical value of a drug, this comprises the assessment of the resistance of a subject to said drug.

The present invention also relates according another embodiment to an array for carrying out the methods of the present invention, said array comprising immobilized proteins, peptides or peptide mimetics comprising one or more phosphorylation sites present in one or more peptide markers. Said proteins, peptides or peptide mimetics are preferably at least 25% of proteins, peptides or peptide mimetics on said array.

More particularly said array comprises immobilized proteins, peptides or peptide mimetics comprising one or more phosphorylation sites as described in detail above representing peptide markers. Additionally said proteins, peptides or peptide mimetics are preferably at least 25%, at least 50%, at least 70%, at least 80%, at least 90% or 100% of the proteins, peptides or peptide mimetics on said array.

The type of arrays to be used according to this embodiment are known in the art and are further detailed above.

The present invention also relates in another embodiment to a computer program product for use in conjunction with a computer having a processor and a memory connected to the processor, said computer program product comprising a computer readable storage medium having a computer program mechanism encoded thereon, wherein said computer program mechanism may be loaded into the memory of said computer and cause said computer to carry out a method according to the present invention.

The present invention further relates to a computer system comprising a processor, and a memory coupled to said processor and encoding one or more programs, wherein said one or more programs instruct the processor to carry out a method according to the present invention.

The present invention further relates in yet another embodiment to a method for determining the effect of a drug on the kinase activity in a sample or predicting the resistance of a subject to a drug, comprising the steps of:
(a) measuring the kinase activity of said sample, in the presence and in the absence of a protein kinase inhibitor, thereby providing the phosphorylation level of one or more phosphorylation sites present in one ore more peptide markers; and,
(b) determining from said phosphorylation levels in the presence and in the absence of a protein kinase inhibitor the effect of said drug on the kinase activity in said sample,
wherein said protein kinase inhibitor mimics the effect of said drug on the kinase activity in said sample.

### EXAMPLES

### EXAMPLE 1 - Bevacizumab (drug) response prediction using Sorafinib inhibition profiles.

A study is performed to generate kinase inhibition profiles from tumor tissues derived from Renal Cell Carcinoma patients treated with the drug Bevacizumab. The tissue is derived from tumor resection performed before the Bevacizumab treatment is started. Bevacizumab is an antibody that blocks the triggering of the Vascular Endothelial Growth Factor Receptors (VEGFR), and thereby blocks the VEGFR signal transduction pathways.

The activity of the targeted VEGFR or VEGFR pathway is assessed in lysates prepared from Bevacizumab responding and non-responding patients. Clinical efficacy is determined using body imaging, thereby monitoring the size reduction of the tumor. In this assessment the lysates are profiled for activities of kinases on a peptide microarray comprising peptide substrates for protein tyrosine kinases. These kinase activity profiling tests are performed both in the presence and absence of Sorafinib, a known protein kinase inhibitor (PKI) of VEGFR kinase and the VEGFR signalling pathway. Sorafinib thereby mimics the effect of Bevacizumab. It is shown that peptides that are inhibited by these VEGFR kinase inhibitors, thus reporting VEGFR activity, are found in the microarray analyses of the Bevacizumab responding patients, and are not inhibited in the analyses of the patients not responding to Bevacizumab (Table 1). Thus a set of peptide inhibition markers is found that can be correlated to the Bevacizumab response. As these inhibition profiles are generated from tumor tissue derived from the patients before the treatment is started, these inhibition profiles are predictive for the clinical response of these patients. In patients with an active VEGFR pathway this drug is clinically active. In patients with no active VEGFR pathway no clinical effects are provided.

**Table 1**

| Patient | Drug | Clinical response | PKI | Test result | Response Prediction |
|---|---|---|---|---|---|
| 1 | Bevacizumab | Responder | Sorafinib | Inhibition | Positive |
| 2 | Bevacizumab | Responder | Sorafinib | Inhibition | Positive |
| 3 | Bevacizumab | Non Responder | Sorafinib | No-Inhibition | Positive |
| 4 | Bevacizumab | Non Responder | Sorafinib | No-Inhibition | Positive |

### EXAMPLE 2 - Bevacizumab (drug) response prediction using inhibition profiles that block alternative (survival) pathways.

A study is performed to generate kinase inhibition profiles from tumor tissues derived from Renal Cell Carcinoma patients treated with the drug Bevacizumab. The tissue is derived from tumor resection performed before Bevacizumab treatment is started. Bevacizumab is an antibody that blocks the triggering of the Vascular Endothelial Growth Factor Receptors (VEGFR), and thereby it blocks the VEGFR signal transduction pathways. In patients with an active VEGFR pathway this drug is expected to be clinically active.

The activity of the alternative pathway is assessed in lysates prepared from Bevacizumab responding and non-responding patients. Clinical efficacy is determined using body imaging, thereby monitoring the size reduction of the tumor. In this assessment the lysates are profiled for activities of kinases on a peptide microarray comprising peptide substrates for protein tyrosine kinases. These kinase activity profiling tests are performed both in the presence and absence of an IGFR kinase inhibitor. If this inhibitor shows an effect on the kinase activities from the tumors, this indicates that this alternative pathway is active in these tumors. It is shown that peptides that are inhibited by this IGFR kinase inhibitor, thus reporting IGFR activity, are found in the analyses of the Bevacizumab responding patients, and are not inhibited in the analyses of the patients not responding to Bevacizumab (Table 2). Thus a set of peptide inhibition markers are found that correlate to Bevacizumab response. As these inhibition profiles are generated from tumor tissue derived from the patients before the treatment is started, these inhibition profiles are predictive for clinical response. Therefore, in patients with no active VEGFR pathway or with an alternative pathway allowing the tumor growth independent of the VEGFR signalling pathway, no clinical effect is obtained.

**Table 2.**

| Patient | Drug | Clinical response | PKI | Test result | Response Prediction |
|---|---|---|---|---|---|
| 1 | Bevacizumab | Responder | IGFR-inhibitor | No-Inhibition | Positive |
| 2 | Bevacizumab | Responder | IGFR-inhibitor | No-Inhibition | Positive |
| 3 | Bevacizumab | Non Responder | IGFR-inhibitor | Inhibition | Positive |
| 4 | Bevacizumab | Non Responder | IGFR-inhibitor | Inhibition | Positive |

## Claims

1. A method for determining the effect of a drug on the kinase activity in a sample, comprising the steps of:
(a) measuring the kinase activity of said sample, in the presence and in the absence of a protein kinase inhibitor, thereby providing a phosphorylation profile of said sample in the presence of a protein kinase inhibitor and a phosphorylation profile of said sample in the absence of a protein kinase inhibitor; and,
(b) determining from said phosphorylation profiles in the presence and in the absence of a protein kinase inhibitor the differential phosphorylation profile, said differential phosphorylation profile indicating the effect of said drug on the kinase activity in said sample,
wherein said protein kinase inhibitor mimics the effect of said drug on the kinase activity in said sample.

2. A method for determining the effect of a drug on the kinase activity in a sample, comprising the steps of:
(a) measuring the kinase activity of said sample, in the presence and in the absence of a protein kinase inhibitor, thereby providing a phosphorylation profile of said sample in the presence of a protein kinase inhibitor and a phosphorylation profile of said sample in the absence of a protein kinase inhibitor; and,
(b) determining from said phosphorylation profiles in the presence and in the absence of a protein kinase inhibitor the differential phosphorylation profile, said differential phosphorylation profile indicating the effect of said drug on the kinase activity in said sample,
wherein said protein kinase inhibitor mimics the effect of said drug on the drug targeted signal transduction pathway in said sample.

3. A method for predicting the resistance of a subject to a drug, comprising the steps of:
(a) measuring the kinase activity of a sample, obtained from said subject, in the presence and in the absence of a protein kinase inhibitor, thereby providing a phosphorylation profile of said sample in the presence of a protein kinase inhibitor and a phosphorylation profile of said sample in the absence of a protein kinase inhibitor; and,
(b) determining from said phosphorylation profiles in the presence and in the absence of a protein kinase inhibitor the differential phosphorylation profile, said differential phosphorylation profile indicating the effect of said drug on the kinase activity in said sample,
wherein said protein kinase inhibitor reports the activity of alternative signal transduction pathways causing tumor growth, which are not affected by the drug, thereby causing resistance.

4. A method for predicting the resistance of a subject to a drug, comprising the steps of:
(a) measuring the kinase activity of said sample, obtained from said subject, in the presence and in the absence of a protein kinase inhibitor, thereby providing a phosphorylation profile of said sample in the presence of a protein kinase inhibitor and a phosphorylation profile of said sample in the absence of a protein kinase inhibitor; and,
(b) determining from said phosphorylation profiles in the presence and in the absence of a protein kinase inhibitor the differential phosphorylation profile, said differential phosphorylation profile indicating the effect of said drug on the kinase activity in said sample,
wherein said protein kinase inhibitor reports the activity of a signal transduction pathway which is not affected by said drug, thereby reporting resistance.

5. Method for predicting the response of a subject to a drug, wherein the effect of a drug on the kinase activity in a sample, obtained from said subject, is determined according to the method of Claims 1 or 2, and wherein the determination of the effect of said drug on the kinase activity in said sample, indicates the response of said subject to said drug.

6. Method according to any of Claims 1 to 5, wherein said phosphorylation profiles comprise the phosphorylation levels of one or more phosphorylation sites present in at least one peptide marker.

7. Method according to any of Claims 1 to 6, wherein said drug acts on receptors and/or acts on the interaction of said receptors with polypeptides.

8. Method according to Claim 7, wherein said receptor is a cell surface receptor and preferably a receptor tyrosine kinase, a G-protein coupled receptor or an integrin receptor.

9. Method according to any of Claims 1 to 8, wherein said protein kinase inhibitor is chosen from the group comprising ErbB2 kinase inhibitor, mimicking the effect of drugs acting on a HER2/Neu receptor; VEGFR kinase inhibitor, mimicking the effect of drugs acting on VEGF or a VEGF receptor; or an EGFR kinase inhibitor, mimicking the effect of drugs acting on an EGF receptor; an IGFR kinase inhibitor, mimicking the effect of drugs on an IGF receptor; an PDGFR kinase inhibitor, mimicking the effect of drugs on an PDGF receptor; or a HGFR (cMET) kinase inhibitor, mimicking the effect of drugs on an HGF receptor.

10. Method according to any of Claims 1 to 9, wherein said differential phosphorylation profile is specific for a certain pathology.

11. Method according to any of Claims 1 to 10, wherein said differential phosphorylation profile is used for diagnostical and/or prognostical purposes, the prediction of the clinical outcome of a therapy, and/or the prediction of side effects and/or toxic effects and/or adverse effects of a therapy.

12. Method according to any of claims 1 to 11, wherein said phosphorylation sites are present on proteins, peptides or peptide mimetics located on a solid support, and preferably a porous solid support or a microarray.

13. Method according to any of Claims 1 to 12, wherein said differential phosphorylation profile predicts the clinical outcome of a drug therapy.

14. Use of the method according to any of the previous claims for assessing susceptibility to a drug or a potential kinase inhibitor of a biological species having a specific disease state or cellular condition.

15. Use of the method according to any of the previous claims for assessing the pharmaceutical or clinical value of a drug.
